# EUROPEAN PATENT APPLICATION

(11) **EP 3 889 964 A1**
(43) Date of publication of application: **06.10.2021**
(21) Application number: 20166965.2
(22) Date of filing: 31.03.2020
(51) Int. Cl.: G16H 15/00, A61F 13/08

(54) **COMPUTER-IMPLEMENTED METHODS AND COMPUTER PROGRAMS FOR PROVIDING ASSISTANCE REGARDING WEARABLE MEDICAL EQUIPMENT**

(71) Applicant: medi GmbH & Co. KG, 95448 Bayreuth (DE)
(72) Inventor: Thelemann, Frank, 95444 Bayreuth (DE)
(74) Representative: Lindner Blaumeier Patent- und Rechtsanwälte

(57) **Abstract**

Computer-implemented method for providing assistance regarding at least one wearable medical equipment (6), in particular a custom-tailored compression garment (8), wherein product specification values (35, 36, 38, 39, 40) for the wearable medical equipment (6), which are used for manufacturing the wearable medical equipment (6) and/or selecting the pre-manufactured wearable medical equipment (6), are determined, characterised in that, for a measurement group (37) of product specification values (38) to be determined by manual measurement performed by a user, a communications process (47) using an, in particular handheld, mobile device (18) is implemented as a measurement assistance function (2), the communication process (47) comprising, for each product specification value (38) in the measurement group (37) and at least one user, at least
- outputting an instruction information guiding the user to at least one measurement position (29) on a body part (13) of a future wearer of the medical equipment (6), the measurement position (29) being associated with the current product specification value (38),
- receiving an input information comprising at least the measured product specification value (38) from the user.

## Description

The invention concerns a computer-implemented method for providing assistance regarding at least one wearable medical equipment, in particular a custom-tailored compression garment, wherein product specification values for the wearable medical equipment, which are used for manufacturing the wearable medical equipment and/or selecting the pre-manufactured wearable medical equipment, are determined. The invention further concerns a corresponding computer program.

Wearable medical equipment comprises, for example, bandages, compression garments and/or orthoses and is used to treat certain medical conditions and/or generally support the wearer, for example during sports or other activities. Such wearable medical equipment is a worn on the body of the user, in particular a body part like a limb. For example, compression stockings provide a certain compression to the lower and, sometimes, also upper leg area. However, for example in the case of back orthoses, in particular wearable spinal orthoses, such medical equipment may also be worn on the torso as a body part.

Product specification, in particular regarding the fitting of the medical equipment, is of high importance to be able to choose or manufacture the correct medical equipment for successfully treating certain conditions. For example, for a compression garment to provide the desired amount of compression to certain anatomical areas, a certain degree of fit of the corresponding compression garment is required. Hence, it is known in the state of the art to measure the body part of a designated wearer of medical equipment, for example a compression garment, such that a fitting standard size may be selected or, preferably, a custom-tailored medical equipment, in particular compression garment, may be provided. The results of measurements on such a body part, for example circumference values, length values and the like, can be understood as product specification values, based on which the right pre-manufactured medical equipment may be chosen and/or production values for producing a custom-tailored medical equipment may be determined, for example for use with a knitting machine or the like.

Regarding such product specification values of a measurement group, that is, product specification values which should be measured at the body part, multiple approaches already exist in the state of the art. For example, a manual measurement by a person, for example staff of a medical store, may be performed, using measurement sheets and/or instructions, for example from literature. After measurement, the measured product specification values may be manually filled into the measurement sheet or the like and the measurement sheet may be sent, in particular faxed, to a provider or manufacturer of medical equipment. However, manually recording measured product specification values has a number of disadvantages, including poor legibility of handwriting, wrong placement of measured values on the measurement sheet, forgetting or overlooking, respectively, of required data, and other human sources of error. Additionally, the data, in particular if sent on paper, has to be manually evaluated by the provider/manufacturer, in particular again manually entered into an ordering system, thus providing additional room for mistakes.

In another approach, digital measurement equipment has been proposed, in particular so-called digital measuring tapes, which allow electronic acquisition of measured product specification values, in particular physical dimensions of the body part. In this manner, data may be automatically electronically forwarded to a provider/manufacturer, such that, for example, the forwarded data may be immediately compared with reference data, for example regarding product selection. For example, EP 1 661 515 A2 discloses a device for electronic acquisition of measurements, in particular for body size measurements of a patient. The device comprises components to digitally scan a measurement tape, which, for example, may have barcodes on it.

However, even such digital acquisition devices still have disadvantages, regarding, for example, wrongly assigning digital acquired data, forgetting and/or overlooking required data and the like.

In a newer approach, the use of 3D scanning devices for scanning the body part, in particular a limb of a patient, have been proposed. Such scanners may be dedicated devices, but may also be implemented using, for example, handheld mobile devices running a corresponding application and evaluating image data of a camera of the handheld mobile device to derive a three-dimensional data set describing at least the surface of the body part, in particular limb. However, such a contactless measurement requires a high amount of room. Additionally, such 3D scanning is not applicable to all patients, in particular not to patients who are confined to bed, have to use a wheelchair or are not able to stand stably. For such patients, manual measurements with all its drawbacks is still required.

US 2017/0082413 A1 discloses a length measuring device and a length measuring system wherein a sensing portion senses an amount of rotation of a first rotating portion and a controller converts the amount of rotation of the first rotating portion into a length measurement. A second rotating portion coiled with the strip by a plurality of times and rotated according to the strip being withdrawn or retracted through an outlet formed in a casing is likewise used for a length measurement.

US 2019/0021634 A1 discloses a method and an apparatus for limb circumference measurement, wherein elastic measurement elements are used to measure circumferences on a plurality of locations of the limb, such that a geometric profile along the limb may be determined.

WO 2009/099587 A2 discloses systems and methods for collecting body measurements, in particular regarding the processing of garment orders. A measurement device having a belt with a first scale of measurement indicia and a strip with a second scale of measurement indicia is used.

US 2006/0031128 A1 discloses a system and associated method of marketing customised articles of clothing. The virtual storefront is accessed via an electronic interface, a selection of a type of clothing article to be customised from the user via the virtual storefront is provided and a selection of at least one feature of the clothing article from the user is provided. An audio/visual query to prompt the user for at least one body measurement is received and, from the user, at least one body measurement response is provided, whereby the at least one body measurement is converted to finished measurements for the clothing article.

It is an object of the current invention to provide a robust and less error-prone method to obtain product specification values of a measurement group, in particular from the wearer himself and/or medical staff, in particular for patients that may not be measured using contactless scanning systems.

This object is achieved by providing a method and a computer program according to the independent claims. Advantageous embodiments are described in the dependent claims.

In a computer-implemented method for providing assistance regarding at least one wearable medical equipment, in particular a custom-tailored compression garment, wherein product specification values for the wearable medical equipment, which are used for manufacturing the wearable medical equipment and/or selecting the pre-manufactured wearable medical equipment, are determined, for a measurement group of product specification values to be determined by manual measurement performed by a user, a communication process using an, in particular handheld, mobile device is implemented as a measurement assistance function, the communication process comprising, for each product specification value in the measurement group and at least one user, a communication subprocess with at least
- outputting an instruction information guiding the user to at least one measurement position on a body part of a future wearer of the medical equipment, the measurement position being associated with the current product specification value,
- receiving an input information comprising at least the measured product specification value from the user.

Throughout this description, medical equipment may comprise one piece of medical equipment or a set of multiple pieces of medical equipment, the pieces serving a common task. Examples for such medical equipment comprise, in particular, bandages, compression garments, and/or orthoses. The method may, preferably, concern custom-tailored compression garments, which may then be manufactured using the product specification values.

The, preferably handheld, mobile device, which may, for example, be a mobile phone, in particular a smartphone, or a tablet, generally comprises a processor, a storage means, an output means and an input means. In preferred embodiment, in particular with the mobile device being a mobile phone, such as a smartphone, and/or a tablet, the mobile device may comprise a touch display as at least one combined output and input means. Preferably, the mobile device may have a computer program according to the invention stored on its storage means. Such a computer program may be an application program, in particular an "app".

Basically, the invention provides a digital assistant for performing measurements, in particular in an interactive communication process. The communication process comprises communication subprocesses for each product specification value, which may be different for different users, as further explained below. Each subprocess, in turn, comprises at least outputting an instruction information guiding the user to at least one measurement position on a body part of a future wearer of the medical equipment, the measurement position being associated with the current product specification value, and receiving an input information comprising at least the measured product specification value from the user. During each communication subprocess, at least one instruction information is output, which guides the user to at least one measurement position, at which the current product specification value, to which the communication subprocess refers, may be measured.

In this manner, the user, which may be medical staff, for example in a medical store, but also in preferred embodiments the future wearer themselves, is guided through the measurement process step-by-step. The danger of forgetting or overlooking product specification values is thus reduced. Furthermore, time investment may also be reduced. Sources of errors, in particular regarding the user and also the manufacturer/provider, if manual rework is necessary, may be, at least partly, eliminated. The provision of instruction information to support users in finding measurement positions and/or generally perform the measurement allows less experienced persons to also perform the measurements.

In preferred embodiments, the instruction information is derived from a rule set describing the relative location of the measurement position to at least one anatomical feature. The rule set is based on actual physical circumstances regarding the body part, such that the user may orient himself using anatomical, locatable anatomical features, in particular landmarks, from which the user is guided to the relevant measurement position by the instruction information, for example by defining certain distances relative to the anatomical feature, certain paths regarding or defined by anatomical features, and the like. In the example of a limb, where circumference values are to be determined at certain measurement positions, a distance in a longitudinal direction of the limb from an anatomical feature may, for example, be used. For example, in a leg, two centimeters above the ankle may be at least one instruction information. Rules may also be on multiple anatomical features, for example finding the middle between two anatomical features and then moving in a longitudinal direction for a certain distance. In such cases, rule may define multiple guidance steps, as further described below.

In this context, at least one rule of the rule set may define an order of the multiple product specification values of the measurement group, wherein the communication subprocesses for each product specification value are performed in this order. For example if the measurement positions are defined by a standard, such as the RAL standard in Germany, the measurement positions may be ordered in a distal or proximal direction along the limb as a body part.

In summary, the method according to the invention allows accurate configuration of wearable medical equipment, in particular compression garments, for a body part, in particular a limb, of a patient, also for patients, which may not be measured using contactless systems. However, the advantages of contactless measuring systems are provided nonetheless, in particular no wrong assignment of measured product specification values to measurement positions, no forgetting of product specification values, no problems regarding legibility of handwriting, reduced time investment, less sources of error, and the like.

In concrete embodiments, the product specification values of the measurement group may comprise at least one length value and/or at least one circumference value of the body part, in particular the limb. That is, product specification values of the measurement group may, in particular, relate to the geometry of the body part. However, since this geometry may be variable according to the current condition/state of the body part, it is, of course, also possible to measure such length values and/or circumference values depending on such a condition/state. Bringing about this condition and/or state may be part of at least one instruction information. For example, circumference values may be measured for both an uncompressed limb and a compressed limb (skin value/tension value). The skin value usually describes the circumference of the limb without any applied compression and is thus equivalent to a base circumference value. The tension value describes the circumference of the limb with the wearable medical equipment, in particular the compression garment, applying a desired compression. In this context, but also generally, it may be advantageous that at least two product specification values sharing all of their at least one associated measurement position share a communication process, in particular a pair of a skin value and a tension value. In this manner, the user is only guided to each measurement positions once, where they may measure both the skin value (which may also be termed uncompressed circumference value) and the tension value (which may also be called tight measurement value). For the tension value, instruction information may also comprise instructions on setting the compression.

In an especially preferred embodiment, the instruction information is at least partly output acoustically, in particular using a speaker of the mobile device, and/or at least partly visually, in particular using a display of the mobile device, and/or the input information is at least partly received acoustically, in particular using a microphone and/or a speech analysis unit of the mobile device, and/or at least partly using an electronic pen, in particular interacting with a touch display of the mobile device. Preferably, at least contactless input of measured product specification value and further information is implemented in a contact-free manner, in particular as speech input and/or handwriting analysis using an electronic pen. Advantageously, the mobile device does not have to be contacted by human skin for documentation and/or ordering the medical equipment, resulting in hygienic improvements. It is particularly preferred to have at least partly acoustic output of instruction information as well as speech input to the mobile device, such that the user can pay his whole attention to the measurement process and is not required to check a visual output means, in particular a touch screen or other display, regularly.

In a further advantageous embodiment, the communication process, in particular each of the communication subprocesses, is interactively dynamically adaptable according to received input information. The communication process and/or at least a part of the communication subprocesses may preferably comprise at least one question-answer process. In this manner, true interactive communication is provided, such that the mobile device provides the capability for a dialog with the user, in particular by speech output and input. This allows the user, for example, to request hints and/or tips regarding a current measurement and/or locating the measurement position. Sophisticated guiding techniques may be employed, simplifying the process for the user and leading to more accurate measurement results.

In a concrete embodiment, the instruction information may be derived from a rule set describing the relative location of the measurement position to at least one anatomical feature, wherein at least a part of the rules of the rule set comprise multiple guidance steps, wherein each communication subprocess comprises communication steps, in particular outputting guidance information, for each guidance step, wherein, in particular, each communication step is ended when input information describing the successful performance of the guidance step by the user is received. In the case that the user is unsuccessful in performing the guidance step, for example, an alternate, refined instruction information for the guidance step may be output in a repetition of the communication step. In this manner, users may be guided to measurement positions and an accurate measurement in a step-by-step process, which is less error-prone.

It is, however, also advantageously conceivable that, if an input information comprising a help request is received, the guidance information regarding the help request is retrieved from a help database and/or generated by an artificial intelligence interaction algorithm and output to the user. In particular, in the case of a help request, the guidance step sequence may be paused and/or reset. In this manner, hints and tips, in particular regarding the measurement positions, may be provided in a help subfunction.

Preferably, further, the communication process may be interrupted due to input information requesting a chat function. Such a chat may be provided with support staff as a chat partner, but also with a chat bot, for example based on an artificial intelligence interaction algorithm. In this manner, the chat function may also be incorporated into the measurement assistance function, such that further assistance and support may be provided, leading to more information provided to the user and thus more accurate, error-free measurements.

As already indicated, in preferred embodiments, the measurement positions may be defined according to at least one standard, in particular the RAL. Often, measurement positions which are relevant for certain treatments by wearable medical equipment are pre-defined in standards, such that those measurement positions may also form the base for specifying medical equipment such that a successful treatment is achieved by custom-tailoring the respective medical equipment or selecting the correct medical equipment, in particular from a number of pre-manufactured sets of medical equipment in different standard sizes.

In especially preferred embodiments, the measurement assistance function may comprise multiple operating modes with at least partly different communication processes and/or rules associated with each operating mode, wherein an operating mode is chosen depending on an experience information describing the experience of the user regarding the measurement of the product specification values. That is, the communication process may be custom-tailored to the experience of the respective user, such that an adequate amount of guidance is provided, while not unnecessarily annoying the user. Preferably, the experience information may, for example, be input or taken from a user profile, however, it is also possible to determine or modify the experience information depending on the interaction of the user in the measurement assistance function and/or on further information regarding the user, in particular feedback information regarding medical equipment already produced and/or selected based on product specification values measured and/or selected by the respective user. If, for example, multiple help requests are entered by the user and/or, in particular in the case of multiple guiding steps, steps often have to be repeated and/or described in more detail, automatical evaluation of this input behaviour may lead to the conclusion that the user is less experienced. However, in particular if the user repeatedly measures product specification values of the measurement group, for example being staff of a medical store, experience information may also be derived regarding the results of previous measurements, for example feedback information, in particular the number of complaints and the like. As will be further discussed below, the mobile device may also comprise a camera, such that, if the measurement process is visible in pictures or videos acquired by the camera, image analysis may also be employed to derive experience information. For example, certain gestures and/or behaviours may be detected and/or time measurements may be performed, also without using the camera, for example from outputting the instruction information to receiving the input information.

Preferably, the operating modes may be chosen from the group comprising a beginner mode, a normal mode, and an expert mode. While, in a beginner mode, the user may be guided to the measurement position using multiple, thoroughly explained guidance steps and thus communication steps, these steps may be abbreviated and/or combined in the normal mode, while in the expert mode, for example, the measurement position may simply be named and a corresponding input information regarding the product specification value requested. In summary, in this embodiment, operating modes for a beginner, an advanced user and a professional user (expert) may be provided, and, accordingly, instruction information is output guiding the user to measurement positions, preferably using a respective rule set. In this manner, measurements may not only be performed by the future wearer himself, in particular as a beginner, but also by less qualified medical staff, for example in medical stores.

A further, especially preferred embodiment provides that, for at least a part of the received product specification values of the measurement group, a plausibility check using a body part information describing typical ranges of such product specification values, in particular relative to at least one other product specification value, is performed. Such a plausibility check allows for including knowledge regarding the physical background of the measurement and the body part, such that the product specification values of the measurement group may more reliably be determined. In particular, the plausibility check may comprise comparisons with at least one threshold value and/or at least one already measured and input product specification value. For example, tension values should be equal or less than corresponding skin values and/or the slope along a limb or other body parts may also fall within certain boundaries or follow a typical course. Such plausibility checks may be based on empirical examinations such that, for example, thresholds and/or other comparison values may describe a typical relative and/or absolute variability of the product specification values in a relevant population.

However, in preferred embodiments, the plausibility check may further comprise employing an artificial intelligence plausibility algorithm. The artificial intelligence plausibility algorithm, whose parametrisation implicitly comprises a body part information which may also have been used as training data for the artificial intelligence plausibility algorithm. This algorithm may also have been trained regarding feedback data describing shortcomings of already produced and/or selected medical equipment, thus detecting patterns of product specification values often leading to complaints.

It is noted that the plausibility check may also take into account rules of the rule set and/or even be determined according to additional rules, in particular plausibility rules, which may be included in the rule set such that only one rule set is required. The rule set then, as one data construct like a database, describes the physical reality/background of the body part and allows, in addition to guidance of the user to measurement positions, for validating measured product specification values regarding conformity with physical reality, including variations.

In preferred embodiments, the instruction information may at least partly comprise at least one image and/or image sequence and/or interactive visualisation of the body part. For example, the general location of the anatomical feature may be indicated in a, for example schematical, depiction of the body part, as well as the relative location of the measurement position. Additionally, for example in an animated sequence, it may be illustrated how to arrive at the measurement position starting from the anatomical feature. Interactive visualisations may allow for manipulating the displayed image and/or image sequence, for example on a touch screen, such that, for example, if an anatomical feature is chosen and/or interacted with, further information regarding the anatomical feature may be output and/or more detailed zoomed-in views of an area the user has interacted with may be displayed. Such images and/or image sequences and/or interactive visualisations allow for the user to more interactively find measurement positions and/or accurately comprehend and be able to reproduce steps in localising the measurement position. It is noted, as will be further discussed below, that the at least one image and/or image sequence and/or interactive visualisation of the body part may comprise and/or even be based on an actual picture of the body part taken by the camera of the mobile device, which has been analysed using principally known image evaluation techniques to, for example, locate anatomical features and/or determine certain dimensions of the body part, on which the actual visual instruction information may be based.

Also generally, in preferred embodiments, the mobile device may have a camera, wherein at least one picture of the body part is taken by the camera, wherein the at least one picture is evaluated to derive at least one wearer-specific instruction information and/or an action information rating a measurement action of the user, when the measurement action and/or a result of the measurement action is visible in the at least one picture, and/or to determine or modify at least one body part information used in a plausibility check as described above. Pictures, that is photographic images, acquired by using the camera of the mobile device may be advantageously used in three different approaches in the method according to the invention. In a first approach, as already described above, actual photographic pictures of the body part may be used to enhance and improve visual instruction information, in particular if such instruction information comprises at least one image and/or image sequence and/or interactive visualisation of the body part. This provides intuitive insight into the actual physical reality of the body part and accordingly improves the guidance for the user.

In a second approach, pictures of the body part acquired using the camera may also be used to analyse measurement actions of the user, if the measurement action and/or the result of the measurement action is visible in the at least one picture. In particular, the user, in particular in the case of a beginner/novice user may be encouraged to position the mobile device such that his measurement actions may be imaged. Such pictures, which may even form a video of the measurement action, can be analysed as principally known in the art and rated, in particular by comparing a user-chosen measurement position to a measurement position determined by evaluation of pictures of the body part and/or by comparing with other parameters describing reference measurement actions, in particular reference movements and/or reference states. An experience information may be used as described above and/or to provide additional guidance/help, in particular in the form of refined instruction information, such that the user may, for example, re-perform the measurement with higher quality and/or hints and/or tips may be provided for future measurement actions. In this manner, the user may be, so to say, trained while using the measurement assistance function.

In a third approach, pictures taken by the camera may also be used to determine or modify at least one body part information used in a plausibility check as described above. For example, principally known image analysis algorithms may be used to determine geometrical information regarding the body part, for example its shape, such that measured and input product specification values of the measurement group may be checked on whether they comply with this body part information. In this manner, an improved plausibility check may be provided.

In especially preferred embodiments, additional to the measurement assistance function, a product properties assistance function to determine further product specification values of a non-measurement group is used. The product specification values of the non-measurement group may, for example, comprise a type of the medical equipment and/or a compression profile of the compression garment and/or at least one material of the medical equipment and/or a weaving and/or knitting method for the medical equipment, in particular the compression garment, and/or a configuration of the medical equipment, as well as further properties. Generally, if a product properties assistance function is additionally provided, all product specification values may be acquired in one go, in particular using one and the same application on the mobile device. This simplifies compilation of all product specification values required, such that production and/or selection of the medical equipment may ensue. However, preferably additionally, the measurement assistance function and/or the product properties assistance function may further comprise at least one additional communication process to acquire at least one wearer-information describing at least one characteristic of the wearer. Wearer information may, for example, comprise the weight of the wearer and/or the age of the wearer and/or the body-mass-index (BMI) of the wearer. Of course, additional wearer information may also be used, for example gender, conditions to be treated and the like.

Product specification values of both groups and/ wearer information may, preferably, synergistically be used. For example, in a preferred embodiment, the measurement group is compiled or chosen depending on at least one product specification value of the non-measurement group and/or at least one wearer information. For example, the type of medical equipment to be specified may determine the body part to be measured and relevant measurement positions, while also wearer information may influence the choice of product specification values of the measurement group. If, for example, product specification values of the non-measurement group specify that the custom-tailored compression garment for a leg is to be produced, measurement positions on the leg according to RAL may be chosen. A condition to be treated may on the one hand be used to determine if, where and possibly under which conditions tension values need to be measured.

On the other hand, in further preferred embodiments, at least one product specification value of the non-measurement group may be determined or adapted depending on at least one measured product specification value of the measurement group and/or at least one wearer information and/or at least one proposal value for at least one product specification value of the non-measurement group may be determined depending on at least one measured product specification value of the measurement group and/or at least one wearer information, wherein the at least one proposal is output for at least one user to select. For example, depending on a resulting shape of the medical equipment, in particular compression garment, it can be determined whether the custom-tailored knitted medical equipment should be produced by flat knitting or circular knitting. If, for example, the measured product specification values, which describe the body part, in particular its shape and/or its compressibility, indicate certain conditions, certain properties of the wearable medical equipment, which are associated with the condition, may be proposed or chosen. In some cases, characteristics or conditions of the measured body part may also lead to proposing another medical equipment category. That is, measured and input product specification values may also be evaluated, in particular in conjunction with wearer information, to determine certain characteristics and/or conditions of the body part, with which certain product specification values of the non-measurement group and/or proposals therefore are associated.

In concrete embodiments, a body part health information may be derived from measured product specification values of the measurement group and/or wearer information, wherein at least one product specification value or a proposal value therefore is determined to yield a medical equipment, in particular a compression garment, suitable for treating a health condition of the body part described by the body part health information.

In embodiments, at least one artificial intelligence algorithm, in particular the artificial intelligence interaction algorithm and/or the artificial intelligence plausibility algorithm, may be further trained for at least one product specification value determination process according to provided outcome and/or feedback information. In other words, communication processes and/or plausibility checks may be adapted based on outcome and/or feedback information, such that, in particular, complaints can be reduced.

In an especially preferred embodiment, after production or selection of the medical equipment, at least one use assistance function may be provided at the mobile device. Such a use assistance function may also be termed, depending on whether the medical equipment is custom-tailored, that is, specifically produced and/or selected, in particular from a group of standard sizes, post production function or post selection function. In this manner, a computer program, in particular an application comprising all three assistance functions, that is, the measurement assistance function, the product properties assistance function and the use assistance function, provides all necessary interaction with, in particular, the wearer and/or optionally medical staff with respect to the wearable medical equipment. In this manner, a holistic approach is sought providing all necessary and required assistance functions regarding wearable medical equipment in single application for mobile devices, in particular smartphones. It has been shown in the course of the current invention that this holistic approach is, independently of the concrete implementation of the measurement assistance function, already very advantageous, as will be further elaborated below.

The inventive method for providing assistance regarding product specification values may be embedded into a method for producing or selecting a wearable medical equipment for a body part of a wearer, comprising automatically performing the steps of the assistance method discussed above, whereafter the medical equipment is automatically produced by a medical equipment production apparatus, in particular a knitting machine, or selected using the at least one product specification value. Of course, all features and remarks discussed regarding the assistance method according to the invention also apply to the production or selection method according to the invention.

The invention also concerns a computer program which performs the steps of an assistance method according to the invention when the computer program is executed on a mobile device. The computer program may, for example, be stored on an electronically readable storage medium, in particular a non-transitional electronically readable storage medium. On the electronically readable storage medium, control information comprising at least one computer program according to the invention is stored, such that, when the electronically readable storage medium is used in a mobile device or the computer program is installed from the storage medium into the mobile device, the mobile device is configured to perform the steps of an assistance method according to the invention. Of course, all explanations and remarks regarding the assistance method regarding the invention accordingly apply to the computer program according to the invention.

In a particularly preferred embodiment, the computer program may comprise, additional to the measurement assistance function, the product properties assistance function and at least one use assistance function. In this manner, the holistic approach already mentioned above can also be implemented for the computer program according to the invention.

As this holistic approach is also applicable independently of the concrete realization of the measurement assistance function, a computer-implemented method for providing assistance regarding at least one wearable medical equipment, in particular custom-tailored compression garment, on an, in particular handheld, mobile device, is also considered advantageous, wherein, to simplify the following discussion, the method of claim 1 will in the following be termed first assistance method, while the additional embodiments here will be termed second assistance method. Of course, the second assistance method may preferably comprise the first assistance method, that is, the measurement assistance function of the second assistance method may be implemented as discussed above.

According to a first aspect of the second assistance method, a computer-implemented method for providing assistance regarding at least one wearable medical equipment, in particular a custom-tailored compression garment, on an, in particular handheld, mobile device is provided, wherein at least three different assistance functions are provided, wherein a part of the assistance functions concern the determination of product specification values for the wearable medical equipment, which are used for manufacturing the wearable medical equipment or selecting the pre-manufactured wearable medical equipment, the assistance functions comprising
- a measurement assistance function to determine product specification values of a measurement group requiring a manual measurement performed by a user,
- a product properties assistance function to determine further product specification values of a non-measurement group, and
- a use assistance function, by which assistance regarding the wearable medical equipment after its production or selection is provided.

As already discussed, since the second assistance method comprises the measurement assistance function as well as the product properties assistance function, all features and advantages described regarding the first assistance function may accordingly be applied here.

The second assistance method implements a holistic approach to assistance regarding wearable medical equipment, providing all necessary assistance functions in a single application and thus the basis for synergistic, advantageous use of data over all these assistance functions. By using the measurement assistance function to assist measurements and the product properties assistance function to determine further product specification values, the wearable medical equipment can be sufficiently specified and configured regarding the future wearer, that is, enough data may be provided to produce or select the wearable medical equipment. Further assistance is provided after the wearer begins to use the garment by the use assistance function, which may also be termed post production assistance function or post selection assistance function, respectively.

Regarding alternate implementations of the measurement assistance function, at least optionally, a three-dimensional dataset of the body part, in particular a limb, may be acquired using the mobile device which, in this case, also has a camera. Such an approach, in which the mobile device is effectively used as a 3D scanning device, has already been proposed in the state of the art. However, in preferred embodiments, both options exist such that, in particular regarding patients whose condition does not allow the three-dimensional scan, assistance according to the first assistance method according to the invention may be provided. Multiple measurement approaches may hence be available in the measurement assistance function. Three-dimensional scanning may, of course, also be only applied to a part of the product specification values of the measurement group. Generally speaking, in these cases, measurement may also comprise acquiring and evaluating a 3D data set using the mobile device for at least some medical equipment.

In a second aspect of the second assistance method according to the first aspect, preferably, in addition to the product specification values, at least one wearer information, in particular regarding a health condition relating to the use of the medical equipment, may be received and/or derived by analysis of the product specification values and stored for use by the use assistance function. That is, data already acquired during product specification may be further used later by the use assistance function. Of course, additionally or alternatively, wearer information may also be received later on, for example during use. In preferred embodiments, the at least one wearer information may comprise an activity index of the wearer regarding the medical equipment and/or the body part. Such an activity index may, for example, be a measure of physical activity of the wearer, in particular regarding the body part.

In a third aspect of the second assistance method according to the first and second aspects, the post-production assistance function may comprise at least one therapy controlling subfunction outputting and/or receiving therapy information to and/or from the wearer. In a concrete, fourth aspect relating to the third aspect, the therapy controlling subfunction comprises monitoring the use and/or therapeutic effect of the medical equipment, in particular by requesting input of therapy information and/or analysis information, in particular comprising a user-performed measurement matching at least one measurement in the measurement assistance function, and/or by analysing the input analysis information and/or analysis information derived from at least one picture taken with a camera of the mobile device and/or comparing current analysis and/or therapy information to prior analysis and/or therapy information and/or product specification values acquired in the measurement assistance function. In the use assistance function, a therapy controlling subfunction may monitor the use and/or the therapeutic effect of the medical equipment. This may be implemented by requesting input of therapy information and/or analysis information from the user. In concrete embodiments, the user may be requested to perform a measurement matching at least one measurement in the measurement assistance function, for example to determine a decrease in swelling or the like. Of course, additional or as an alternative to such analysis information, general therapy information may also be requested from the user, for example the results of examinations by a physician or other medical personal. The therapy controlling subfunction may analyse the input analysis information and/or therapy information in the cause of monitoring, while it is also possible that analysis information is derived by the therapy controlling subfunction itself, in particular from at least one picture taken with a camera of the mobile device. Principally known image analysis algorithms, as already discussed above, may also be employed here. The therapy controlling subfunction may, in the course of monitoring the use and/or therapeutic effect of the medical equipment, also compare current analysis and/or therapy information to prior analysis and/or therapy information and/or product specification values acquired in the measurement assistance function. If, for example, a large circumference due to swelling at a leg or arm has been measured using the measurement assistance function, which is now treated by a corresponding medical equipment, for example a custom-tailored compression garment, the corresponding circumference may be re-measured in the scope of the use assistance function to detect a therapeutic effect of the medical equipment, in particular compression garment.

In especially preferred embodiments, when comparing with measured product specification values, a fit monitoring regarding the medical equipment may be performed. That is, it can be checked whether the medical equipment still fits the body part and is thus able to provide the desired treatment effect. In particular, as will be further discussed later on, replacement of the wearable medical equipment can be triggered.

In a fifth aspect of the second assistance method, referring to the third and fourth aspect, output therapy information may comprise instructions of use for the medical equipment and/or skin care information and/or information regarding additional therapy actions to be performed by the wearer of the medical equipment. The variety of information regarding correct use of the wearable medical equipment, in particular at least partly derived from the monitoring action of the therapy controlling subfunction, may be output to the user, in particular hints and tips on how the use of the medical equipment may be improved and/or supplemented. In this regard, an application comprising the assistance function becomes sort of a companion providing interactive assistance and helpful information.

In a sixth aspect of the second assistance function, referring to the third to fifth aspects, the definition and production or selection of at least one wearable medical equipment for further therapy may be triggered based on the evaluation of therapy information, in particular in conjunction with patient information. For example, a follow-up product may be specified, in particular at least partly using the measurement assistance function and/or the product properties assistance function. If, for example, therapy information, in particular a comparison with measured product specification values, indicates that a certain treatment effect, in particular a certain treatment success, is achieved, the current medical equipment may be replaced by new, better fitting wearable medical equipment. Wearer information, as discussed above, may additionally be taken into account regarding the triggering, for example describing the health condition.

In a seventh aspect relating to the sixth aspect of the second assistance method, at least one product specification value for the further therapy medical equipment may be determined based on therapy information and/or product specification values of the at least one previously-used medical equipment. That is, for example for areas of the body part where no change is apparent, product specification values of the previously-used medical equipment may also be used for further therapy medical equipment, while other product specification values may be derived from therapy information, in particular re-measured values. In this manner, synergistically, multiple medical equipments are adapted to one another and the product specification process is simplified and, in particular, shortened. In especially preferred embodiments, a complete, specified product may be proposed to the wearer in the use assistance function.

In preferred embodiments of the second assistance method, therapy information, in particular in conjunction with wearer information according to the second aspect and/or regarding multiple sets of medical equipment, is analysed with respect to diagnosis correlation and/or secondary medical conditions. The set of medical equipment is to be understood as at least one piece of medical equipment serving a common purpose. For example, the multiple sets of medical equipment may comprise at least one bandage and/or at least one compression garment and/or at least one orthosis. By analysing the therapy information, in particular together with wearer information, secondary medical conditions can be identified and/or different diagnoses may be correlated. Preferably, the analysis covers multiple sets of medical equipment used by the wearer. In this manner, also medical staff may be assisted regarding further treatment of the wearer.

In an eighth aspect of the second assistance method, generally, the use assistance function may comprise a reminder subfunction reminding the wearer of events associated with the medical equipment, in particular therapy actions to be performed. If, for example, a time schedule regarding therapy actions is provided, reminders can be output on the mobile device, such that therapy actions or other processes and/or events are not missed by the wearer. In other words, the use assistance function may comprise a reminder system in the form of the reminder subfunction.

In this respect, the time and content of the reminders may be user-defined and/or received as input, but are preferably at least partly automatically determined, in particular depending on at least one property of the medical equipment, in particular expressed by at least one product specification value, and/or wearer information according to the second aspect. For example, for certain types of medical equipment, certain time schedules may be pre-defined regarding therapy actions and/or other events, for example also comprising re-adjustment events and the like.

In a ninth aspect of the second assistance method, a communication interface connecting at least one assistance function to an electronic health record of a wearer of the at least one medical equipment is used. In this manner, synergetic data fusion with electronic health records (EHR) can be achieved to further improve assistance, treatment, and functionality of the second assistance method.

For example, in a tenth aspect, referring to the ninth aspect, at least one information received or determined by the at least one assistance function connected to the electronic health record is stored in the electronic health record and/or at least one information extracted from the at least one electronic health record is evaluated by the at least one assistance function connected to the electronic health record, in particular for determining at least one product specification value and/or at least one therapy information. Information received or determined by an assistance function connected to the electronic health record can be made available in the electronic health record, in particular for further evaluation, in particular with additional health data of the electronic health record. However, advantageously, at least one information extracted from the at least one electronic health record may be evaluated in the connected assistance function, such that, for example, less product specification values need to be entered by the user, in particular wearer, and/or proposals for product specification values may be determined and presented as an output for selection by a user. Extracted information may, for example, comprise at least one wearer information as discussed above, and/or information regarding a prescription to be treated. Sometimes, electronic health records may even comprise information regarding the wearable medical equipment, for example in the case of a prescription of the medical equipment.

In an eleventh aspect, referring to the ninth or tenth aspect, at least one electronic prescription is triggered and/or implemented by the at least one assistance function connected to the electronic health record. For example, if, as discussed above, secondary medical conditions are detected and/or a currently used medical equipment should be replaced by a further therapy medical equipment, a respective prescription may be automatically triggered if the respective assistance function, in this case in particular the use assistance function, is connected to the electronic health record of the wearer. For example, the prescription may be forwarded into the electronic health record and/or to a medical professional identifiable from the electronic health record, such that it can be further handled.

In a twelfth aspect of the second assistance method, generally, the use assistance function may comprise at least one feedback subfunction for submitting feedback regarding the produced or selected medical equipment to the producer and/or a seller of the medical equipment. Such feedback information may, in particular, comprise a complaint, but also positive feedback, such as treatment effectiveness and the like.

In a thirteenth aspect, referring to the twelfth aspect, in concrete embodiments, at least one, in particular annotated, picture taken with a or the camera of the mobile device may be submitted as part of the feedback information. That is, in the case of complaints or other feedback, an explanatory picture, in particular having an annotation, may be sent, in particular showing the current state of the wearable medical equipment, for example showing the cause for complaint. This simplifies feedback and complaint evaluation at the manufacturer or provider of the wearable medical equipment.

It is noted that therapy information and the like may, of course, also form part of the feedback information.

In a fourteenth aspect of the second assistance method, at least one artificial intelligence function of at least one assistance function may be trained based on training information obtained from at least one other assistance function. For example, if feedback information, therapy information and the like is collected by the use assistance function, such information may advantageously be used to train artificial intelligence functions/algorithms used in the measurement assistance function and/or the product properties assistance function, for example the already-mentioned artificial intelligence interaction algorithm or artificial intelligence plausibility algorithm. Of course, also data transfer in the opposite direction is possible, such that an artificial intelligence function of the use assistance function is trained using information obtained by any of the two specification assistance functions. In this manner, further advantageous synergistic effect is achieved.

In a fifteenth aspect of the second assistance method, an , in particular anonymised, medical equipment information, in particular comprising feedback information and/or therapy information, may be provided to an external data base, in particular for big-data evaluation and/or artificial intelligence function training. Preferably anonymised medical equipment information, for example comprising product specification values and/or therapy information and/or feedback information, may be provided to a data base for statistical evaluation processes, such that general trends and/or patterns regarding product properties, use of medical equipment, complaint issues and the like can be detected and used for further improvement of wearable medical equipment, treatment and/or assistance methods.

Preferably, in a sixteenth aspect of the second assistance function, at least one interactive help assistance function, in particular comprising a question interpreter, may be provided as an additional assistance function. For example, such a question interpreter may allow a user, in particular the wearer, to get specifically the information he is looking for. In some embodiments, the question interpreter may be implemented as a chat bot, in particular an artificial intelligence chat bot, such that the user/wearer may be guided to a precise formulation of the question and corresponding data may be output, after being, for example, retrieved from a help data base or the like, which can be accessed by the interactive help assistance.

In a seventeenth aspect, a computer program may be provided, which performs the steps of a second assistance method according to any of the preceding first to sixteenth aspects, when it is executed on a system comprising the mobile device. In particular in the case that the assistance functions are all accessed by the (future) wearer of the wearable medical equipment, the system may comprise only the mobile device, running the computer program as an application which implements all the assistance functions. All comments and remarks regarding the second assistance method accordingly apply to the computer program according to the seventeenth aspect.

Further details and advantages of the current invention will become apparent from the following description of exemplary embodiments taken in conjunction with the drawing, in which
- Fig. 1: is a principle drawing showing the structure and purpose of a method/computer program according to the invention,
- Fig. 2: shows an environment for using a measurement assistance function,
- Fig. 3: shows an example of visual output information,
- Fig. 4: is an exemplary flow chart of a communication process,
- Fig. 5: explains using the measurement assistance function and the product properties assistance function to determine a full set of product specification values,
- Fig. 6: is a schematical drawing explaining operating modes, and
- Fig. 7: illustrates possible subfunctions and results of a use assistance function.

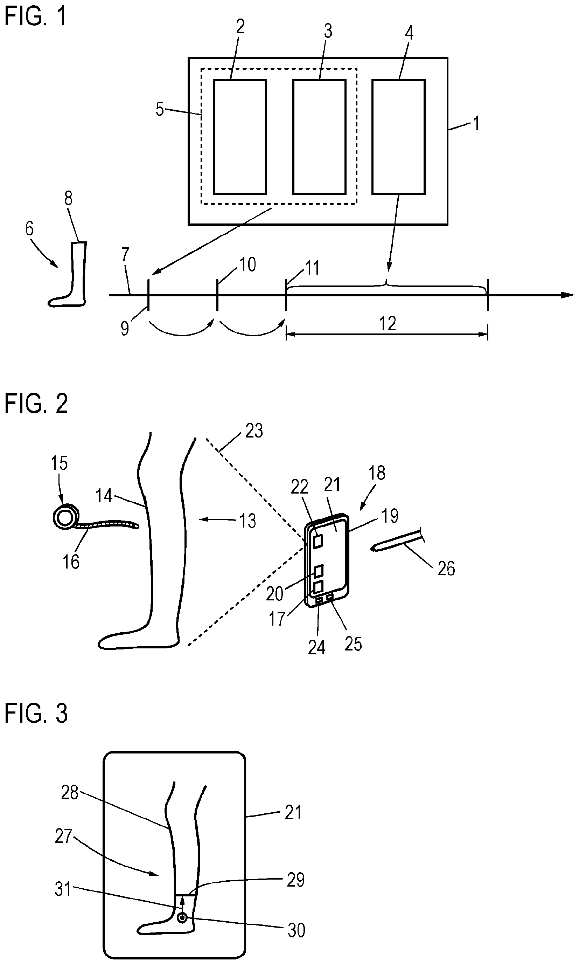

Fig. 1 shows the general structure of preferred embodiments of an inventive computer-implemented method for providing assistance regarding at least one wearable medical equipment. As can be seen, a computer program 1 implementing the method comprises three main assistance functions 2, 3, 4, namely a measurement assistance function 2, a product properties assistance function 3 and a use assistance function 4. The system functions 2 and 3 can be grouped into a group 5 of functions for determining product specification values of a measurement group (measurement assistance function 2) and of a non-measurement group (product properties assistance function 3).

The computer program 1 is, in such preferred embodiments, an application (app) for a handheld mobile device, in particular a mobile phone (smartphone) or a tablet. This application serves as a companion over the lifetime of the wearable medical equipment 6, including design/ selection phase, in fig. 1 indicated by timeline 7. While the wearable medical equipment 6 may be, for example, a bandage or an orthosis, in the preferred embodiment described below, a compression garment 8 will mostly be used as an example.

The measurement assistance function 2 and the product properties assistance function 3 of the group 5 are both used at a time-point 9 corresponding to the action of compiling a complete set of product specification values, which allow, at a time-point 10, the production/manufacturing of a custom-tailored medical equipment 6 or a selection of a pre-manufactured medical equipment 6 from among standard sizes. The medical equipment 6 is then provided to a future wearer, who starts using the medical equipment 6 at a time-point 11. The use assistance function 4 provides assistance to the wearer during the time period 12 in which the medical equipment 6 is used.

The measurement assistance function 2 is used for determining a measurement group of product specification values, which require manual measurement performed by a user, in particular the future wearer himself. To this end, a communication process is implemented.

A corresponding scenario is shown in fig. 2. In this case, the body part 13 which should be measured is a limb, in particular a leg 14. The manual measurements may be taken using a standard measurement tool 15, for example a measurement tape 16. In this case, assistance is provided by running the computer program 1, which may be stored in a corresponding storage means 17, on a handheld mobile device 18, in this case a smartphone 19, which, in addition to the indicated storage means 17, comprises a processor 20, a touch display 21 (touchscreen) as combined input and output means, a camera 22 for acquiring pictures in a field of view 23, a microphone 24 as further input means, and a speaker 25 as further output means. An electronic pen 26 is further provided for non-contact use of the touch display 21.

As can be seen from fig. 2, the mobile device 18 is positioned such that the body part 13 is located inside the field of view 23 during the measurement process, such that pictures of the body part 13 and/or the user performing the measurement can be taken.

For each product specification value of the measurement group, which may, for example, comprise circumference values and/or length values of the body part, associated measurement positions are provided. For manual measurement at such a measurement position, a communication subprocess of the communication process is performed, wherein at least one instruction information guiding the user to the measurement position is output, wherein, in this case, both the touch display 21 and the speaker 25 are used as output means for the instruction information.

An exemplary visual instruction information on the touch display 21 is shown in fig. 3. The image 27 comprises a depiction 28 of the leg 14, which may be based on a picture taken by the camera 22. Since, in this embodiment, the instruction information is derived from a rule set describing the relative location of the measurement position 29 to at least one anatomical feature 30, both the measurement position 29 as well as the anatomical feature 30 may be included in the image 27, in this case additionally showing an instructing arrow 31 which may, for example, also have an annotation describing a relative distance to be covered.

The measurement positions 29 may be defined by a standard, for example German RAL standard, which contains measurement positions 29 for different body parts 13, for example hands, arms, feet and legs 14.

In not yet laid-open European Patent Application EP 19167008.2, such rule sets have already been proposed to automatically locate measurement positions in three-dimensional data sets of a limb. In the method disclosed there, anatomical features of the limb are located and rules are applied to automatically find respective measurement positions. Such rules may also, in the context of the current invention, be used to provide instructions to a user, since anatomical features are easier to locate as such for the user than measurement positions and, once the relative location of the measurement position to the anatomical feature is known, corresponding guidance can be provided, in particular, at least in some cases, in multiple guidance steps.

Generally speaking, a rule of the rule set may describe how a measurement position may be derived from the position of an anatomical feature. While the position of an anatomical feature may, in some cases, already define at least one measurement position, regarding other measurement positions, physical and/or anatomical considerations may lead to more complex relationships, for example certain offset to at least one position of an anatomical feature, relative positions regarding multiple anatomical features and the like. For example, in the case of a leg 14, a first measurement position may be determined as three centimetres above the malleolus, a second measurement position may be determined as two centimetres below the lower edge of the patella and/or five centimetres below the middle of the patella, and the like. Those rules may be determined in empirical studies of a plurality of persons or otherwise.

As can be seen in the example of fig. 3, the anatomical feature 30 is the ankle, from which a certain length upwards a circumference at measurement position 29 shall be measured.

It is noted that, additionally or alternatively to the visual instruction information described with respect to fig. 2, animated versions and/or sequences of images/pictures, in particular videos, and the like may also be used. The visual instruction information on the touch display 21 is also interactive, such that, for example, interaction of the electronic pen 26 with an anatomical feature may lead to information about the anatomical feature to be displayed, a zoom to be performed, or the like.

Once the user has followed the instructions, found the measurement position 29 and measured the product specification value, the product specification value is input and thus received in the mobile device 18. This is performed contactless, either, preferably, by using the microphone 24 or by using the electronic pen 26. Speech reception and analysis is especially preferred, since the user can fully concentrate on the measurement task. In particular a combination of speech output of the instruction information, which may be supported by visual output as described above if needed, with speech input is advantageous, such that the user can be in speech dialogue with the measurement assistance function 3.

It should further be noted that some product specification values of the measurement group, for example a skin value and a tension value, may share at least one measurement position, such that the communication subprocess is preferably performed only once for both these product specification values, such that the user has to be guided to each measurement position only once.

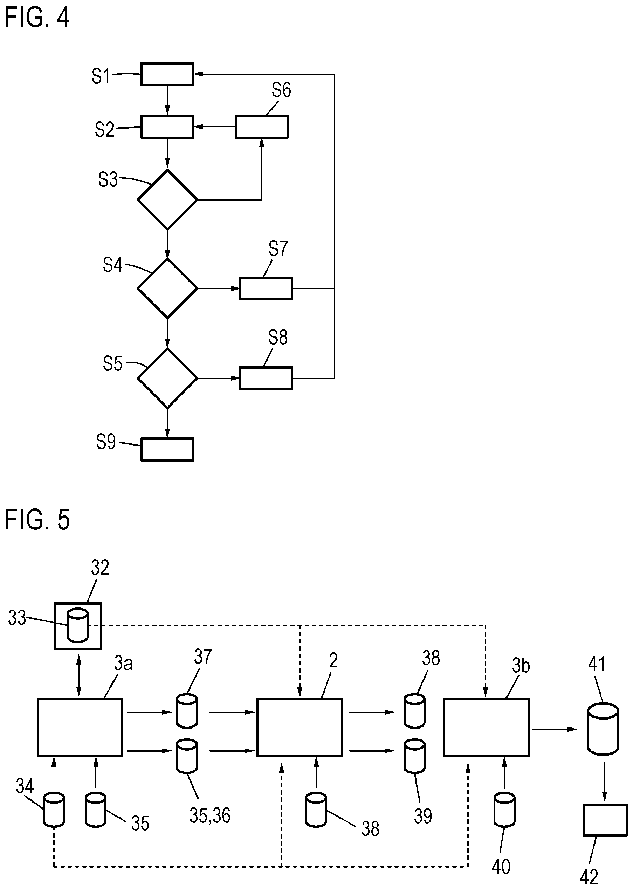

Generally, the communication process is interactively dynamically adaptable according to received input information, in particular may comprise at least one question-answer process. A communication subprocess of a concrete embodiment is illustrated in fig. 4. For a first product specification value of the measurement group, in a step S1, instruction information is output. In this example, for at least a part of the product specification values of the measurement group, the rules of the rule set comprise multiple guidance steps, such that the communication subprocess may comprise multiple communication steps before the actually measured product specification value is received.

Thus, instruction information for a first of the at least one guidance step (and later on further guidance steps) is output in the first occurrence of step S1.

In a step S2, input information is received, as described, and evaluated in steps S3, S4 and S5.

If, in step S3, it is determined that the input information describes unsuccessful performance of the guidance step, a help request, or some predefined interaction with the image 27 or sequence of images displayed on the touch display 21, in a step S6, correspondingly updated output information is output, for example a refined, more accurate instruction information in the case of a help request or unsuccessful performance of the guidance step, or by providing a detailed view or additional information regarding the feature which was interacted with. For example, when interacting with the anatomical feature 30, that is the ankle, in fig. 3, information about locating the ankle may be additionally displayed and/or a more detailed view of the ankle may be provided. In some embodiments, pictures, in particular a video, acquired by the camera 22 may also be evaluated regarding a measurement action performed by the user, such that hints and tips regarding actual mistakes may be output. However, as should be noted at this point, in preferred embodiments, pictures of the body part 13 taken by the camera 22 are most preferably evaluated to derive wearer-specific instruction information, where applicable.

After step S6, input information is again received in step S2.

In step S4, it is checked whether the input information comprises a chat request, such that, in a step S7, for example, a chat window may be opened in which interactive communication with a chat bot or support staff may be initiated.

In step S5, it is checked whether the input information contains the product specification value to be measured. In other words, it can be evaluated if further guidance steps follow. If this is the case, the instruction information is updated accordingly in step S8 and the previous steps are repeated for the next guidance step, such that an additional communication step results.

If, in step S5, it has been determined that the measured product specification value has been received, in a step S9, a plausibility check for the product measurement value may be performed. It is noted that step S9 may also be performed at a later stage, for example, when multiple product specification values have been measured.

The plausibility check uses body part information and may comprise comparisons with at least one threshold and/or already measured and input product specification values. For example, as can be seen from figures 2 and 3, in the case of a leg 14, the body part 13 has a certain shape such that, in particular by empirical studies, maximum variations and/or ranges for certain product specification values and/or their differences can be derived and used to perform the plausibility check. For example, regarding circumferences, the gradient (or slope) between two points should lie in a certain range or should have a certain algebraic sign. This prior knowledge regarding the physical reality of the body part, in particular the body part information, is used to prevent erroneous measurements and/or inputs. The plausibility check may also advantageously evaluate pictures taken by the camera 22 and/or use evaluation results of such pictures derived in other steps. Such pictures often show the general structure and geometry of a body part in a sufficiently accurate way for plausibility considerations. Such pictures may hence also form part of the body part information.

It is noted that, if plausibility rules are used in the plausibility check, these rules may also be included in the rule set described above, such that only one rule set regarding the body part is required and may, for example, be provided as a database containing all-purpose body part information.

In some embodiments, in step S9, an artificial intelligence plausibility algorithm may be employed, which may also have been trained regarding feedback data describing shortcomings of already produced and/or selected medical equipment 6, such that the artificial intelligence plausibility algorithm may detect patterns of product specification values often leading to complaints and providing corresponding output and/or correction.

If the plausibility check in step S9 fails, the user is notified accordingly using the speaker 25 and/or the touch screen 21, such that the input information may be corrected and/or the measurement re-performed and/or, in particular in the case of preventing complaints, adapted, in particular according to a certain proposal.

It is noted at this point that, in the measurement assistance function 2 and/or in the product properties assistance function 3, wearer information may be additionally received from the user or from another source, wherein the wearer information may determine some product specification values and/or product specification values to be used; however, it is also possible that product specification values of the non-measurement group influence product specification value of the measurement group and vice versa, as will be further explained below with regard to fig. 5. In fig. 5, the measurement assistance function 2 is performed interleaved between two stages 3a, 3b of the product properties assistance function 3. In a first stage or segment 3a of the product properties assistance function 3, the product properties assistance function 3 connects to an electronic health record 32, from which a first wearer information 33 may be requested and received. Further wearer information 34 may be input after a correspondingly output request by the user. Wearer information may, for example, comprise a gender of the wearer and/or the age of the wearer and/or a body-mass-index (BMI) of the wearer, but may also relate to medical conditions to be treated by the wearable medical equipment 6.

Additionally, in the stage 3a, some product specification values 35 of the non-measurement group may be received as input information from the user. It is noted that further product specification values or proposals therefore may, in stage 3a, be automatically determined from the wearer information 33, 34, sometimes in conjunction with input product specification values 35. That is, the output of stage 3a regarding the non-measurement group may not only comprise the user-received product specification values 35, but also additional product specification values 36 of the non-measurement group derived from the received product specification values 35 and/or the wearer information 33, 34.

However, in this embodiment, the measurement group 37, that is, the measurement specification values to be requested, may also be compiled or chosen depending on at least one product specification value 35, 36 of the non-measurement group and/or at least one wearer information 33, 34. In an example, if a custom-tailored compression garment for a leg 14 is to be specified, the product specification values of the measurement group 37 and the respective measurement positions may be defined according to a standard, for example RAL. It is noted at this point that, if a rule set is used, the rule set may also comprise an order in which the product specification values are to be measured, which may also be provided as input to the measurement assistance function 2.

It is noted that, in some embodiments, wearer information 33, 34 may be used to refine and personalise instruction information regarding the wearer, as indicated by the dashed lines. In any case, measured product specification values 38 for the measurement group 37 are received from the user, as described above. However, as should be noted, for example depending on wearer information 33, 34, some product specification values of the measurement group 37 may also be automatically determined in rare cases.

Some product specification values 39 of the non-measurement group may also be automatically determined based on received product specification values 38 of the measurement group 37 and/or wearer information 33, 34, such that they do not need to be entered in the second stage 3b of the product properties assistance function 3. It is, in some embodiments, also possible that proposals for the user to select may be output for at least some product specification values of the non-measurement group. In a concrete example, it may be derived from the user-receipt product specification values 38 of the measurement group 37 whether flat knitting or circular knitting is a preferred production method.

In some embodiments, it is also possible to derive a body part health information from measured and user-received product specification values 38 of the measurement group 37, wherein at least one product specification value or a proposal value therefore may be determined such that a medical equipment 6 suitable for treating a health condition of the body part 13 described by the body part health information is provided. In this evaluation process, additionally, wearer information 33, 34 may be preferably used. For example, another medical equipment category may be recommended, in particular even replacing a user-received product specification value 35 or derived product specification value 36 of the first stage 3a.

In stage 3b, analogously to stage 3a, product specification values 40 of the non-measurement group that are still missing may be requested and received correspondingly from the user. Hence, as an output of stage 3b, a complete set 41 of product specification value 35, 36, 38, 39, 40 is provided, such that, in a step 42, a corresponding medical equipment 6 may be manufactured or selected. In the case of manufacturing a custom-tailored wearable medical equipment 6, in particular a compression garment 8, production values for controlling a garment production apparatus, in particular a knitting machine, may be automatically derived and the garment 8 may be automatically produced.

It is noted that, while speech recognition and acoustical dialogue with the user are very advantageous during the measurement process, of course, also the input of product specification values 35, 40 of the non-measurement group may be performed voice-controlled.

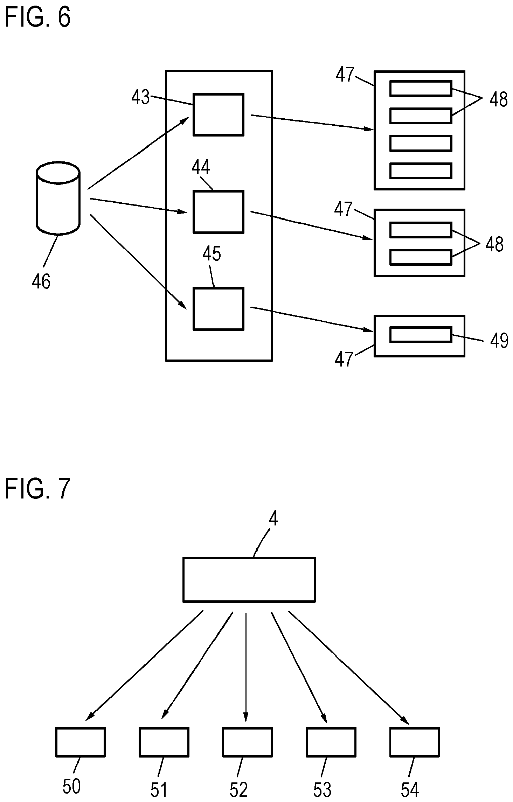

As shown in fig. 6, the communication process may be performed in multiple operating modes 43, 44, 45, depending on an experience information 46 describing the experience of the user. The experience information 46 may, for example, be at least partly input by the user. It is, however, preferred to at least partly derive the experience information 46 from the interaction of the user with the computer program 1, in particular the measurement assistance function 2. Additionally, feedback information regarding medical equipment 6 already produced and/or selected based on product specification values 38 measured and/or selected by the respective user, may be taken into account. It is especially preferred to also evaluate pictures, in particular a video, taken by the camera 22 during measurement action of the user, if these measurement actions are depicted in the at least one picture.

Depending on the experience information 46, a beginners operating mode 43, a normal operating mode 44 and an expert operating mode 45 are chosen. In the operating mode 43, a communication subprocess 47 of the communication process may comprise multiple, thoroughly explained communication steps 48 associated with guidance steps. In the normal mode, the number of communication steps 48 in the communication subprocess 47 has already been considerably reduced, while in the expert mode, the communication subprocess 47 may be reduced to a single communication step 49. In a concrete example, when measuring circumference values at a certain RAL measurement position, in the beginners operating mode 43, the user may be guided step-by-step from the anatomical feature 30 to the measurement position 29. In the expert operating mode 45, the RAL measurement position may simply be named.

Finally, fig. 7 illustrates possible subfunctions and results of the use assistance function 4. For example, the use assistance function 4 may comprise a therapy controlling subfunction 50, which may preferably monitor the use and/or therapeutic effect of the medical equipment. In particular, the user, in particular the wearer, may be prompted to repeat at least one measurement of at least one product specification value 38 of the measurement group 37 for comparison, such that, for example, a reduction of swelling can be observed. The therapy controlling subfunction may also analyse pictures from the camera 22. This analysis, overall, may also trigger the definition (specification) and production or selection of at least one wearable medical equipment 6 for further therapy, for example, if the fitting of the previous medical equipment 6 is no longer accurate enough. Regarding the further therapy wearable medical equipment 6, of course, the measurement assistance function 2 and the product properties assistance function 3 may, again, be used.

The therapy controlling subfunction 50 may also comprise outputting information, for example instructions for use of the medical equipment 6, skin care information and/or information regarding additional therapy actions to be performed by the wearer.

A reminder subfunction 51 may be used to remind a user, in particular the wearer, of events associated with the medical equipment, for example certain therapy actions to be performed, like a gymnastic exercise or checking compression levels. The use assistance function may also use a communication interface already used by the product properties assistance function 3, as described above, to access the electronic health record 32, such that information may be exchanged, in particular regarding therapy, and/or even an electronic prescription may be triggered and/or implemented.

The use assistance function 4 may further comprise a feedback subfunction 53, such that feedback information, in particular a complaint, may be submitted to a manufacturer (producer) and/or a provider (seller) of the medical equipment 6.

Feedback information may, preferably, comprise at least one, in particular annotated, picture taken with the camera 22 of the mobile device 18.

Finally, the use assistance function 4 may comprise an interactive help assistant 54, which may preferably comprise a question interpreter to provide general support and assistance regarding the medical equipment 6.

## Claims

1. Computer-implemented method for providing assistance regarding at least one wearable medical equipment (6), in particular a custom-tailored compression garment (8), wherein product specification values (35, 36, 38, 39, 40) for the wearable medical equipment (6), which are used for manufacturing the wearable medical equipment (6) and/or selecting the pre-manufactured wearable medical equipment (6), are determined, **characterised in that**, for a measurement group (37) of product specification values (38) to be determined by manual measurement performed by a user, a communications process using an, in particular handheld, mobile device (18) is implemented as a measurement assistance function (2), the communication process comprising, for each product specification value (38) in the measurement group (37) and at least one user, a communication subprocess (47) with at least
- outputting an instruction information guiding the user to at least one measurement position (29) on a body part (13) of a future wearer of the medical equipment (6), the measurement position (29) being associated with the current product specification value (38),
- receiving an input information comprising at least the measured product specification value (38) from the user.

2. Method according to claim 1, **characterised in that** the product specification values (38) of the measurement group (37) comprise at least one length value and/or at least one circumference value of the body part (13) and/or that at least two product specification values (38) sharing all of their at least one associated measurement positions (29) share a communication subprocess (47), in particular a pair of a skin value and a tension value.

3. Method according to claim 1 or 2, **characterised in that** the instruction information is at least partly output acoustically, in particular using a speaker (25) of the mobile device (18), and/or at least partly visually, in particular using a display (21) of the mobile device (18), and/or that the input information is at least partly received acoustically, in particular using a microphone (24) and/or a speech analysis unit of the mobile device (18), and/or at least partly using an electronic pen (26), in particular interacting with a touch display (21) of the mobile device (18).

4. Method according to one of the preceding claims, **characterised in that** the communication process is interactively dynamically adaptable according to received input information.

5. Method according to claim 4, **characterised in that** the instruction information is derived from a rule set describing the relative location of the measurement position (29) to at least one anatomical feature (30), wherein at least a part of the rules of the rule set comprise multiple guidance steps, wherein each communication subprocess (47) comprises communication steps (48), in particular outputting guidance information, for each guidance step, wherein, in particular, each communication step (48) is ended when input information describing the successful performance of the guidance step by the user is received.

6. Method according to claim 4 and 5, **characterised in that**, if an input information comprising a help request is received, a guidance information regarding the help request is retrieved from a help database and/or generated by an artificial intelligence interaction algorithm and output to the user.

7. Method according to one of the preceding claims, **characterised in that** the measurement assistance function (2) comprises multiple operating modes (43, 44, 45) with at least partly different communication processes and/or rules associated with each operating mode (43, 44, 45), wherein an operating mode (43, 44, 45) is chosen depending on an experience information (46) describing the experience of the user regarding the measurement of the product specification values (38).

8. Method according to one of the preceding claims, **characterised in that**, for at least a part of the received product specification values (38) of the measurement group (37), a plausibility check using a body part information describing typical ranges of such product specification values (38), in particular relative to at least one other product specification value (38), is performed.

9. Method according to one of the preceding claims, **characterised in that** the instruction information at least partly comprises at least one image (27) and/or image sequence and/or interactive visualization of the body part (13).

10. Method according to one of the preceding claims, **characterised in that** a mobile device (18) having a camera (22) is used, wherein at least one picture of the body part (13) is taken by the camera (22), wherein the at least one picture is evaluated to derive at least one wearer-specific instruction information and/or an action information rating a measurement action of the user, when the measurement action and/or a result of the measurement action is visible in the at least one picture, and/or to determine or modify at least one body part information used in a plausibility check according to claim 8.

11. Method according to one of the preceding claims, **characterised in that**, additional to the measurement assistance function (2), a product properties assistance function (3) to determine further product specification values (35, 36, 39, 40) of a non-measurement group is used, and/or that the measurement assistance function (2) and/or the product properties assistance function (3) comprises at least one additional communication process to acquire at least one wearer information (33, 34) describing at least one characteristic of the wearer.

12. Method according to claim 11, **characterised in that** the measurement group (37) is compiled or chosen depending on at least one product specification value (35, 36, 39, 40) of the non-measurement group and/or at least one wearer information (33, 34).

13. Method according to claim 11 or 12, **characterised in that** at least one product specification value (35, 36, 39, 40) of the non-measurement group is determined or adapted depending on at least one measured product specification value (38) of the measurement group (37) and/or at least one wearer information (33, 34), and/or at least one proposal value for at least one product specification value (35, 36, 39, 40) of the non-measurement group is determined depending on at least one measured product specification value (38) of the measurement group (37) and/or at least one wearer information (33, 34), wherein the at least one proposal is output for at least one user to select.

14. Method for producing or selecting a wearable medical equipment (6) for a body part (13) of a wearer, comprising automatically performing the steps of a method according to one of the preceding claims, whereafter the medical equipment (6) is automatically produced by a garment production apparatus, in particular a knitting machine, or selected using the at least one product specification value (35, 36, 38, 39, 40).

15. Computer program (1), which performs the steps of a method according to any of the claims 1 to 13 when the computer program (1) is executed on a mobile device (18).

16. Computer program (1) according to claim 15, **characterised in that** it comprises, additional to the measurement assistance function (2), the product properties assistance function (3) and at least one use assistance function (4).
